(19) Europäisches Patentamt
European Patent Office
Office européen des brevets

(11) Veröffentlichungsnummer: **0 124 014**
**B1**

# (12) EUROPÄISCHE PATENTSCHRIFT

(45) Veröffentlichungstag der Patentschrift: **05.10.88**

(51) Int. Cl.⁴: **C 07 D 303/08**, C 07 D 303/04, C 07 D 303/22, C 07 D 301/02 // C07D249/08

(21) Anmeldenummer: **84104333.4**

(22) Anmeldetag: **17.04.84**

## (54) Verfahren zur Herstellung von Oxiranen.

(30) Priorität: **29.04.83 DE 3315510**

(43) Veröffentlichungstag der Anmeldung: **07.11.84 Patentblatt 84/45**

(45) Bekanntmachung des Hinweises auf die Patenterteilung: **05.10.88 Patentblatt 88/40**

(84) Benannte Vertragsstaaten: **BE CH DE FR GB LI**

(56) Entgegenhaltungen:
**EP - A - 0 040 345**
**CH - A - 614 201**

**CHEMISCHE BERICHTE, Band 96, nr. 7, 5. Juli 1963, pp. 1881-1890, WEINHEIM (DE), V. FRANZEN et al.: "Umsetzung von Sulfonium-Yliden mit polaren Doppelbindungen"**
**CHEMICAL ABSTRACTS, Band 96, nr. 13, 29. März 1982, p. 684, Zusammenfassung Nr. 103999u, COLUMBUS OHIO (US), R.M. MUNAVU et al.: "The preparation of trimethylsulfonium bromide and its utility in the synthesis of oxiranes"**

(73) Patentinhaber: **BAYER AG, Konzernverwaltung RP Patentabteilung, D-5090 Leverkusen 1 Bayerwerk (DE)**

(72) Erfinder: **Mohrmann, Karl Heinrich, Dr., Roonstrasse 61, D-5600 Wuppertal 1 (DE)**
Erfinder: **Reiser, Wolf, Dr., Kiebitzweg 12a, D-5600 Wuppertal 1 (DE)**
Erfinder: **Linke, Siegfried W., Dr., Bayer Pharma Korea Ltd., Daehan Building, 10th Floor 75 Seosomondong, Choong-ku Seoul (KR)**
Erfinder: **Zerbes, Rudolf, Dr., In der Beek 26, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft ein neues Verfahren zur Herstellung von bekannten Oxiranen, die als Zwischenprodukte zur Synthese von Verbindungen mit pflanzenwuchsregulierender und fungizider Wirksamkeit verwendet werden können.

Es ist bereits bekannt geworden, dass man Oxirane herstellen kann, indem man Dimethylsulfid mit Methylbromid umsetzt und das dabei entstehende Trimethylsulfoniumbromid anschliessend mit Carbonyl-Verbindungen in Gegenwart eines inerten organischen Lösungsmittels und in Gegenwart einer starken Base, wie Natriumhydrid, Natriumamid oder Kalium-tert.-butylat, zur Reaktion bringt (vgl. Chem. Ber. 96, 1881–1890 (1963) und Kenya J. Sci. Technol.; Ser. A., 1980, 111–115).

Die Umsetzung von Cabonyl-Verbindungen, die in Nachbarstellung zur Carbonyl-Gruppe einen tert.-Butyl-Rest enthalten, wird nach der erwähnten Methode jedoch nicht beschrieben.

Weiterhin ist bekannt, dass sich 2-(4-Chlorphenylethyl)-2-tert.-butyl-oxiran herstellen lässt, indem man aus Dimethylsulfid und Dimethylsulfat zubereitetes Trimethylsulfonium-methylsulfat mit 1-(4-Chlorphenyl)-4,4-dimethyl-pentan-3-on in Acetonitril in Gegenwart von Natriummethylat umsetzt (vgl. EP-A-0 040 345). Die Ausbeuten bei diesem Verfahren sind gut, aber dennoch für praktische Zwecke nicht immer ausreichend.

Es wurde nun gefunden, dass man die bekannten Oxirane der Formel

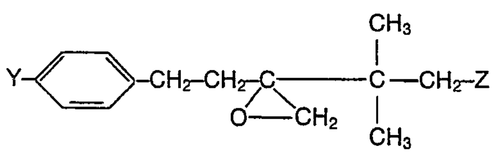 (I)

in welcher

Y für Chlor oder Phenyl steht und

Z für Wasserstoff oder Halogen steht,

erhält, wenn man Dimethylsulfid mit Methylbromid in Gegenwart eines inerten organischen Verdünnungsmittels behandelt und das dabei entstehende Trimethylsulfoniumbromid der Formel

$$(CH_3)_3S^{\oplus}BR^{\ominus} \qquad (II)$$

mit einem Keton der Formel

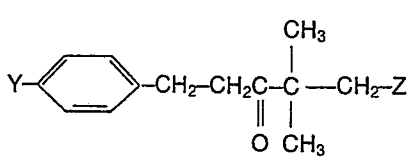 (III)

in welcher

Y und Z die oben angegebene Bedeutung haben,

in Gegenwart einer starken anorganischen oder organischen Base sowie in Gegenwart eines inerten organischen Verdünnungsmittels bei Temperaturen zwischen 0 °C und 60 °C umsetzt.

Es ist äusserst überraschend zu bezeichnen, dass sich Oxirane der Formel (I), z.B. das 2-(4-Chlor-phenyl-ethyl)-2-tert.-butyl-oxiran, nach dem erfindungsgemässen Verfahren in höherer Ausbeute herstellen lassen als nach dem aus der EP-A-0 040 345 bekannten Verfahren, bei dem Dimethylsulfid und Dimethylsulfat als Ausgangsstoffe dienten.

Aufgrund der Lehre, die aus den Artikeln in Chem. Ber. 96, 1881–1890 (1963) hervorgeht, war ferner anzunehmen, dass eine Verzweigung neben der Carbonyl-Gruppe die Umsetzung von Ketonen in die entsprechenden Oxirane behindert.

Das erfindungsgemässe Verfahren besitzt eine Reihe von Vorteilen. So ermöglicht es die Herstellung von Oxiranen der Formel (I) in sehr guten Ausbeuten. Ausserdem sind die Ausgangsstoffe relativ preisgünstig und auch in technischem Massstab verfügbar.

Die nach dem erfindungsgemässen Verfahren herstellbaren Oxirane sind durch die Formel (I) definiert. In dieser Formel steht der Rest Z vorzugsweise für Wasserstoff, Fluor oder Chlor.

Verwendet man bei dem erfindungsgemässen Verfahren neben Dimethylsulfid und Methylbromid das 1-(4-Chlor-phenyl)-4,4-dimethyl-pentan-3-on als Ausgangsstoff und Kalium-tert.-butylat als Base, so kann der Reaktionsablauf durch das folgende Formelschema veranschaulicht werden:

a) $(CH_3)_2S + CH_3Br \longrightarrow (CH_3)_3S^{\oplus} Br^{\ominus}$

b) 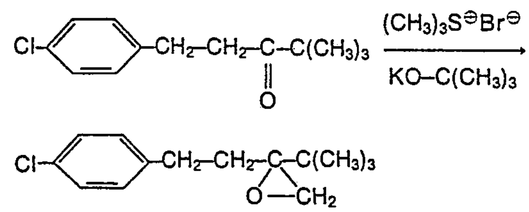


Die bei dem erfindungsgemässen Verfahren als Ausgangsstoffe benötigten Ketone sind durch die Formel (III) definiert. In dieser Formel steht der Rest Z vorzugsweise für Wasserstoff, Fluor oder Chlor.

Die Ketone der Formel (III) sind bekannt (vgl. DE-C-2 201 063, DE-A-2 705 678 und DE-A-2 737 489).

Das bei dem erfindungsgemässen Verfahren weiterhin als Ausgangsstoff benötigte Trimethylsulfonium-bromid der Formel (II) ist ebenfalls bekannt (vgl. Chem. Ber. 96, 1881–1890 (1963) und Kenya J. Sci. Technol.; Ser. A., 1980, 111–115). Es wird bei der obigen Umsetzung in frisch hergestelltem Zustand, gegebenenfalls ohne vorherige Isolierung, verwendet.

Als starke anorganische und organische Basen können bei dem erfindungsgemässen Verfahren vorzugsweise Natriumhydrid, Natriumamid, ausserdem Alkalialkoholate, wie Natriummethylat, Natriumethylat und Kalium-tert.-butylat, und ferner auch Alkalihydroxide, wie Kaliumhydroxid und Natriumhydroxid, verwendet werden.

Als Verdünnungsmittel können bei dem erfindungsgemässen Verfahren sowohl bei der Herstellung des Trimethylsulfonium-bromids als auch bei der anschliessenden Umsetzung dieses Stoffes mit einem Keton der Formel (III) alle inerten organischen Lösungsmittel verwendet werden. Vorzugsweise kommen bei der Umsetzung von Dimethylsulfid und Methylbromid als Solventien Nitrile, wie Acetonitril, oder niedrig siedende Ketone, wie Aceton, in Frage. Bei der anschliessenden Umsetzung des Trimethylsulfonium-bromids mit einem Keton der Formel (III) kommen Nitrile, wie Acetonitril, ferner polare Solventien, wie Dimethylsulfoxid, weiterhin aromatische oder aliphatische Kohlenwasserstoffe, wie Hexan, Benzol, Toluol und Xylol, und ausserdem auch Alkohole, wie Isopropanol und tert.-Butanol, als Lösungsmittel in Betracht. Es können auch Gemische dieser Lösungsmittel verwendet werden.

Die Reaktionstemperaturen können bei der Durchführung des erfindungsgemässen Verfahrens innerhalb eines bestimmten Bereiches variiert werden. Im allgemeinen arbeitet man sowohl bei der Herstellung des Trimethylsulfonium-bromids als auch bei dessen anschliessender Umsetzung mit einem Keton der Formel (III) bei Temperaturen zwischen 0 °C und 60 °C, vorzugsweise zwischen 10 °C und 40 °C.

Das erfindungsgemässe Verfahren wird im allgemeinen bei Normaldruck durchgeführt. Es ist aber auch möglich, bei erhöhtem oder vermindertem Druck zu arbeiten.

Bei der Durchführung des erfindungsgemässen verfahrens setzt man in der ersten Stufe Dimethylsulfid mit einer äquivalenten Menge oder auch mit einem Überschuss an Methylbromid um. In der zweiten Stufe werden die Mengen an Reaktionskomponenten im allgemeinen so gewählt, dass auf 1 Mol an Keton der Formel (III) 1,0 bis 2,0 Mol, vorzugsweise 1,1 bis 1,5 Mol an Trimethylsulfonium-bromid und 1,0 bis 2,0 Mol, vorzugsweise 1,0 bis 1,5 Mol an Base vorhanden sind.

Im einzelnen verfährt man bei der Durchführung des erfindungsgemässen Verfahrens in der Weise, dass man in eine Lösung von Dimethylsulfid in einem organischen Solvens Methylbromid einleitet und dann das entstandene Salz entweder nach vorheriger Isolierung oder aber direkt in ein Gemisch aus Keton der Formel (III) und Base in einem organischen Solvens gibt. Die Aufarbeitung erfolgt nach üblichen Methoden. Im allgemeinen geht man so vor, dass man das Reaktionsgemisch mit einem Oxidationsmittel, wie z. B. einer wässrigen Wasserstoffperoxid-Lösung oder einem Gemisch aus verdünnter, wässriger Natrium- oder Kaliumhypochlorit-Lösung, und ferner mit einem mit Wasser wenig mischbaren Lösungsmittel und Wasser versetzt, die organische Phase abtrennt, wäscht und nach gegebenenfalls vorherigem Trocknen einengt.

Das dabei anfallende Produkt kann zur weiteren Reinigung unter vermindertem Druck destilliert werden.

Die nach dem erfindungsgemässen Verfahren herstellbaren Oxirane der Formel (I) sind wertvolle Ausgangsstoffe zur Synthese von 1-Hydroxyethyl-azol-Derivaten welche hervorragende pflanzenwuchsregulierende und fungizide Eigenschaften besitzen (vgl. EP-A-0 040 345).

So lässt sich beispielsweise das
1-(4-Chlorphenyl)-4,4-dimethyl-3-(1,2,4-triazol-1-yl-methyl)-pentan-3-ol
der Formel

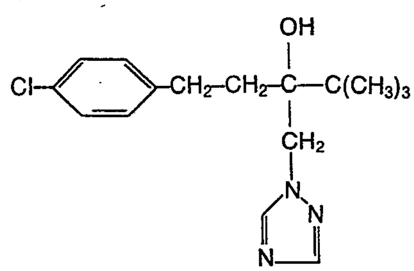

herstellen, indem man 2-(4-Chlor-phenyl-ethyl)-2-tert.-butyl-oxiran mit 1,2,4-Triazol in Gegenwart von Kaliumhydroxid umsetzt. Diese Synthese lässt sich formelmässig wie folgt veranschaulichen:

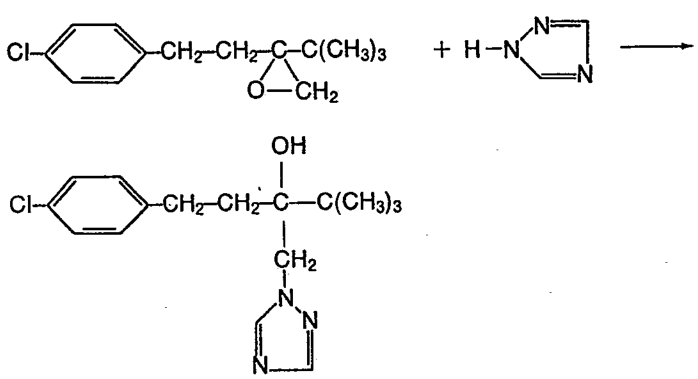

Das erfindungsgemässe Verfahren wird durch die nachfolgenden Beispiele veranschaulicht.

Beispiel 1

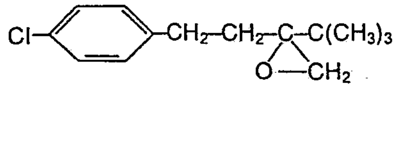

In eine Lösung von 62 g (1 Mol) Dimethylsulfid in 300 ml Aceton wurden bei 20 °C innerhalb von 4 Stunden 95 g (1 Mol) Methylbromid eingeleitet. Man lässt 10 Stunden nachrühren, saugt dann das ausgefallene Trimethylsulfonium-bromid ab und trocknet das Salz bei 30 °C. Auf diese Weise erhält man 115 g (73% der Theorie) an Trimethylsulfonium-bromid.

In ein Gemisch von 22,4 g (0,2 Mol) Kalium-tert.-butylat in 100 ml tert.-Butanol wurden bei Raumtemperatur unter Rühren 22,4 g (0,1 Mol) 1-(4-Chlor-phenyl)-4,4-dimethyl-pentan-3-on gegeben. Danach wurden 23,5 g (0,15 Mol) Trimethylsulfonium-bromid zugesetzt, wobei die Temperatur des Reaktionsgemisches auf 30 °C anstieg. Nach 22-stündigem Nachrühren bei Raumtemperatur wurde ein quantitativer Umsatz erzielt. Der Gehalt an dem gewünschten Endprodukt betrug gemäss gaschromatographischer Analyse 98,8%. Zur Aufarbeitung wurde das Reaktionsgemisch nacheinander mit 200 ml Wasser und 20 ml wässriger Natriumhypochlorit-Lösung versetzt. Danach wurde mit insgesamt 250 ml Ethylenchlorid extrahiert, die organische Phase mit Wasser neutral gewaschen und anschliessend durch Abziehen des Lösungsmittels unter vermindertem Druck eingeengt. Es verblieb ein öliger Rückstand von 21,1 g, der gemäss Gaschromatogramm zu 95,9% aus 2-(4-Chlor-phenyl-ethyl)-2-tert.-butyl-oxiran bestand. Danach errechnet sich eine Ausbeute von 84,8% der Theorie.

Vergleichsbeispiel
Herstellung von 2-(4-Chlor-phenyl-ethyl)-2-tert.-butyl-oxiran der Formel

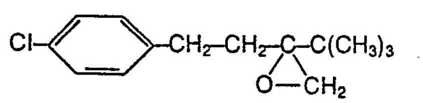

nach bekanntem Verfahren.

Zu einer Lösung von 126 ml (1,33 Mol) Dimethylsulfat in 670 ml Acetonitril wurde unter Rühren eine Lösung von 108 ml (1,47 Mol) Dimethylsulfid in 130 ml Acetonitril getropft. Man liess das Reaktionsgemisch über Nacht stehen und versetzte dann mit 79,2 g (1,47 Mol) festem gepulvertem Natriummethylat, wobei die Temperatur des Reaktionsgemisches auf etwa 20 °C gehalten wurde. Danach wurde eine Lösung von 179 g (0,8 Mol) 1-(4-Chlor-phenyl)-4,4-dimethyl-pentan-3-on in 250 ml Acetonitril zugetropft. Das Reaktionsgemisch wurde 4 Stunden nachgerührt und dann über Nacht stehen gelassen. Anschliessend wurde das Reaktionsgemisch unter vermindertem Druck eingeengt, und der verbleibende Rückstand wurde in Essigester gelöst. Die dabei entstehende Lösung wurde mit Wasser gewaschen und nach dem Trocknen über Natriumsulfat durch Abziehen des Lösungsmittels unter vermindertem Druck eingeengt.

Der verbleibende Rückstand wurde einer Vakuumdestillation unterworfen. Man erhielt auf diese Weise 157 g eines Produktes, das einen Siedepunkt von 102–105 °C bei 0,01 mbar aufwies und das gemäss Gaschromatogramm zu 53% aus 2-(4-Chlor-phenyl-ethyl)-2-tert.-butyl-oxiran bestand. Danach errechnet sich eine Ausbeute von 43% der Theorie.

Beispiel für die Verwendung eines erfindungsgemäss herstellbaren Oxirans zur Synthese eines 1-Hydroxyethylazol-Derivates mit pflanzenwuchsregulierender und fungizider Wirksamkeit

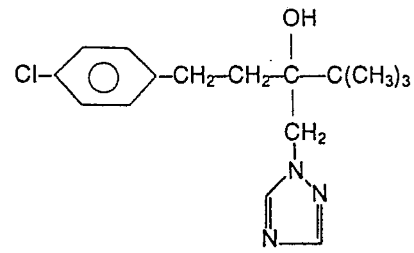

Eine Lösung von 27,1 g (0,1 Mol) eines Produktes, das zu 88% aus 2-(4-Chlor-phenyl-ethyl)-2-tert.-butyl-oxiran bestand, 8,3 g (0,12 Mol) 1,2,4-Triazol und 0,06 g (0,01 Mol) Kaliumhydroxid in 100 ml n-Propanol wurde 30 Stunden auf 95 °C erhitzt. Danach liess man abkühlen und engte das Reaktionsgemisch durch Abziehen des Lösungsmittels unter vermindertem Druck ein. Der verbleibende Rückstand wurde in Toluol aufgenommen, die dabei entstehende Suspension wurde filtriert, und das Filtrat wurde durch Abziehen des Lösungsmittels unter vermindertem Druck eingeengt. Der anfallende Rückstand wurde aus Ligroin umkristallisiert. Man erhielt auf diese Weise 30,6 g eines Produktes, das gemäss HPLC-Analyse zu 67,4% aus 1-(4-Chlor-phenyl)-4,4-dimethyl-3-(1,2,4-triazol-1-yl-methyl)-pentan-3-ol bestand. Danach errechnet sich eine Ausbeute von 67% der Theorie.

**Patentansprüche**

1. Verfahren zur Herstellung von Oxiranen der Formel

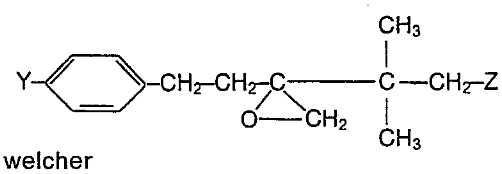 (I)

in welcher
Y für Chlor oder Phenyl steht und
Z für Wasserstoff oder Halogen steht,
dadurch gekennzeichnet, dass man Dimethylsulfid mit Methylbromid in Gegenwart eines inerten organischen Verdünnungsmittels behandelt und das dabei entstehende Trimethylsulfonium-bromid der Formel

$$(CH_3)_3S^{\oplus}Br^{\ominus} \quad (II)$$

mit einem Keton der Formel

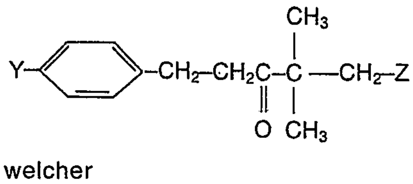 (III)

in welcher
Y und Z die oben angegebene Bedeutung haben,
in Gegenwart einer starken anorganischen oder organischen Base sowie in Gegenwart eines inerten organischen Verdünnungsmittels bei Temperaturen zwischen 0 °C und 60 °C umsetzt.

2. Verfahren gemäss Anspruch 1, dadurch gekennzeichnet, dass man die Umsetzung bei Temperaturen zwischen 10 °C und 40 °C durchführt.

3. Verfahren gemäss Anspruch 1, dadurch gekennzeichnet, dass man in der zweiten Stufe des Verfahrens als organische Verdünnungsmittel Acetonitril, Dimethylsulfoxid, Toluol, Isopropanol und/oder tert.-Butanol einsetzt.

4. Verfahren gemäss Anspruch 1, dadurch gekennzeichnet, dass man als Keton der Formel (III) 1-(4-Chlor-phenyl)-4,4-dimethyl-pentan-3-on einsetzt.

**Claims**

1. Process for the preparation of oxiranes of the formula

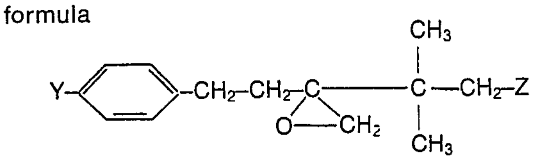 (I)

in which

Y represents chlorine or phenyl and

Z represents hydrogen or halogen,

characterized in that dimethyl sulphide is treated with methyl bromide in the presence of an inert organic diluent, and the trimethylsulphonium bromide produced thereby, of the formula

$$(CH_3)_3S^{\oplus}BR^{\ominus} \quad (II)$$

is reacted with a ketone of the formula

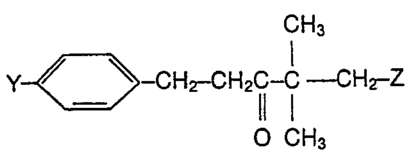 (III)

in which

Y and Z have the meanings indicated above, in the presence of a strong inorganic or organic base and in the presence of an inert organic diluent, at temperatures between 0 °C and 60 °C.

2. Process according to Claim 1, characterized in that the reaction is carried out at temperature between 10 °C and 40 °C.

3. Process according to Claim 1, characterized in that acetonitrile, dimethyl, sulphoxide, toluene, isopropanol and/or tert.-butanol are employed as organic diluents in the second step of the process.

4. Process according to Claim 1, characterized in that 1-(4-chlorophenyl)-4,4-dimethyl-3-pentanone is employed as the ketone of the formula (III).

**Revendications**

1. Procédé de préparation d'oxirannes de formule

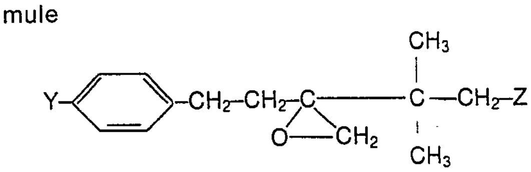 (I)

dans laquelle

Y représente le chlore ou un groupe phényle et

Z représente l'hydrogène ou un halogène,

caractérisé en ce que l'on traite le sulfure de diméthyle par le bromure de méthyle en présence d'un diluant organique inerte, ce qui donne le bromure de triméthylsulfonium de formule

$$(CH_3)_3S^{\oplus}Br^{\ominus} \quad (II)$$

qu'on fait réagir avec une cétone de formule

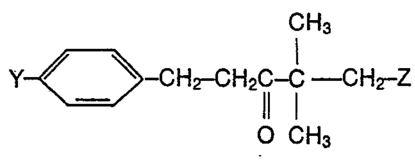 (III)

dans laquelle

Y et Z ont les significations indiquées ci-dessus, en présence d'une base inorganique ou organique forte et en présence d'un diluant organique inerte à des températures de 0 à 60 °C.

2. Procédé selon la revendication 1, caractérisé en ce que l'on effectue la réaction à des températures de 10 à 40 °C.

3. Procédé selon la revendication 1, caractérisé en ce que, dans le deuxième stade opératoire du procédé, on utilise en tant que diluants organiques l'acétonitrile, le diméthylsulfoxyde, le toluène, l'isopropanol et/ou le tert-butanol.

4. Procédé selon la revendication 1, caractérisé en ce que l'on utilise en tant que cétone de formule III la 1-(4-chlorophényl)-4,4-méthyl-pentane-3-one.